**Europäisches Patentamt**

**European Patent Office**

**Office européen des brevets**

(19)

(11) Publication number: **0 183 716**

**B1**

(12) # EUROPEAN PATENT SPECIFICATION

(45) Date of publication of patent specification: **04.01.89**

(51) Int. Cl.⁴: **G 01 N 1/30,** G 01 N 33/52, G 01 N 33/48

(21) Application number: **85902018.2**

(22) Date of filing: **12.04.85**

(86) International application number:
**PCT/EP85/00162**

(87) International publication number:
**WO 85/05180 21.11.85 Gazette 85/25**

(54) **IDENTIFICATION OF MYELOBLASTS AND OTHER IMMATURE GRANULOCYTIC CELLS.**

(30) Priority: **27.04.84 US 604741**

(43) Date of publication of application:
**11.06.86 Bulletin 86/24**

(45) Publication of the grant of the patent:
**04.01.89 Bulletin 89/01**

(84) Designated Contracting States:
**BE CH DE FR GB IT LI NL**

(56) References cited:
**GB-A-2 074 749**
**GB-A-2 095 402**
**GB-A-2 116 712**

(73) Proprietor: **Kass, Lawrence**
**1939 Ridge Road**
**Hinckley Ohio 44233 (US)**

(72) Inventor: **Kass, Lawrence**
**1939 Ridge Road**
**Hinckley Ohio 44233 (US)**

(74) Representative: **Kador & Partner**
**Corneliusstrasse 15**
**D-8000 München 5 (DE)**

Courier Press, Leamington Spa, England.

## Description

Background of the invention

Cytochemistry of blood cells began with Ehrlich in 1879. Using a triacid staining mixture composed of orange G, methyl green and acid fuchsin, Ehrlich obtained differential coloration of the various types of white blood cells. The names he applied to these cells such as neutrophils, eosinophils and basophils are in established use today. Their study forms the foundation of modern hemotology.

Soon after Ehrlich's basic work, other types of stains were discovered in conjunction with efforts to demonstrate malarial parasites. The earliest of these were the stains of Malachowski and Romanowsky in 1891. The so-called Romanowsky mixture of eosin, methylene blue, azure and eosinates soon supplanted Ehrlich's original triacid stain. At present, modifications of the Romanowsky stain, such as Wright's or Giemsa's stain, are used widely in hemotology for blood cell identification.

Early in the history of blood cell identification, it became apparent that the differentiation and identification of the early, immature or primitive precursor cells in blood and bone marrow created difficulties when using "Romanowsky" type staining mixtures. To circumvent this problem, cytochemical stains were developed. Cytochemistry represents biochemistry based on studies at microscopic and submicroscopic levels. Applied to blood and bone marrow cells, cytochemistry identifies mature cells and their primitive or immature precursors on the basis of a characteristic property, such as a unique enzyme or cellular metabolite, rather than on physical features such as size, shape or color.

Among the earliest cytochemical stains to be applied to the problem of blood and bone marrow cell identification was the peroxidase stain. Used initially as a measure of the oxidizing ferment in vegetables, the peroxidase staining method was applied to identification of blood cells about 75 years ago. Soon, it became apparent that some cells contained peroxidase activity (granulocytes) and other cells did not (lymphocytes). Not long afterwards, the peroxidase stain was applied to the study of immature or primitive cells in acute leukemia. For the first time it became possible to distinguish immature or primitive granulocytic cells, called myeloblasts, from immature lymphoid cells, or lymphoblasts.

This distinction could be achieved on the basis of the detection of myeloperoxidase activity in myeloblasts but not in lymphoblasts.

In the customary practice of cytochemical staining of myeloperoxidase requires the addition of a color forming compound when oxidized, an exogenous chromogen, commonly benzidine or o-tolidine, coupled with an addition of an oxidizing agent, illustratively hydrogen peroxide.

In the case of benzidine, for example, it is a suspected carcinogen and is of potential hazard to the user. The reaction product is unfortunately unstable and fades over a period of time. Furthermore, some leukemic myeloblasts presently identified as myeloblasts with monoclonal antibodies, do not contain demonstrable peroxidase activity.

Over the past several decades, other tests have been devised as possible alternatives to the myeloperoxidase reaction, because of the limitations of this reaction as noticed.

First of these alternative tests was the Sudan black B stain. Sudan black B is known as Cl solvent black 3 or Cl 26150. Applied to fixed preparations of blood and bone marrow cells, Sudan black B stains lipids in the granules of granulocytic cells at all stages of their development. The stain is weakest when used in conjunction with immature granulocytic cells, like myeloblasts and promyelocytes. It is strongest in mature granulocytic cells such neutrophils, eosinophils and basophils. Although useful in the laboratory for distinctive identifications between leukemic myeloblasts leukemic lymphoblasts. Sudan black B stain shows poor localization in granular structures. Furthermore, there is considerable nonspecific background precipitation of the dyestuff in many of the contemporary methods for its use.

Along with the development of Sudan black B as an alternative to the traditional myeloperoxidase reaction, a specific esterase enzyme was identified as unique to cells of the granulocytic series. Using naphthol-ASD-chloroacetate as the substrate and a sensitized dye like Fast blue BBN as the indicator, the specific esterase stain demonstrates granulocytic properties in immature cells like myeloblasts in acute myeloblastic leukemia or in granulocytic sarcoma (chloroma). This stain and method has several shortcomings. These include the need for an exogenous substrate, use of a sensitized unstable dye coupler, imprecise localization of the reaction product in granules, need for a nuclear counterstain, prolonged incubation period to perform the test, and considerable background precipitation of the coupler, making it difficult to distinguish between artifact and reaction product.

As a result of the shortcomings of the specific esterase reaction, other stains have been developed as alternatives. Used as direct stains applied to fixed preparations of blood and bone marrow cells, these later stains were simpler in application than the traditional stains and have produced comparable results.

Complexities associated with the specific esterase reaction tests led to the discovery that chlorozol black E (direct black 38, Cl 30235) in which both primary granules or lysosomes, and secondary granules or specific granules have been distinguished and demonstrated in neutrophilic granlocytic cells on the basis of identifiable differences.

Using Saturn blue; Cl 42045, also known as acid blue 1; similar differences in primary and secondary granules have been noted.

Using Niagara sky blue 6B; Cl 24410, also known as direct blue 1; identifiably differential coloration of primary and secondary granules provided an advance in the art.

With the acid dye known as sulfonaphthyl red, an acid dye not identified by Colour Index number, both primary and secondary granules are stained red.

So far as presently known, no dyestuff has heretofor been suggested or reported which selectively and preferentially differentially stains only primary granules or lysosomes in dried and/ or fixed cells of the neutrophilic granulocytic series including myeloblasts, promyelocytes, myeolocytes, bands and neutrophils.

Heretofor, all of the useful stains have been either acid dyes or direct or solvent dyes and non have been basic cationic dyes. None of the foregoing have been selective, since they stain both primary and secondary granules, producing in some instances color differences.

Detailed description of the invention

The dyestuff of this invention as used in Claim 1 and applied in the method of Claim 2 is the sole basic cationic dyestuff presently known in the art which preferentially and selectively stains primary granules or lysosomes in the cells of the neutrophilic granulocytic series.

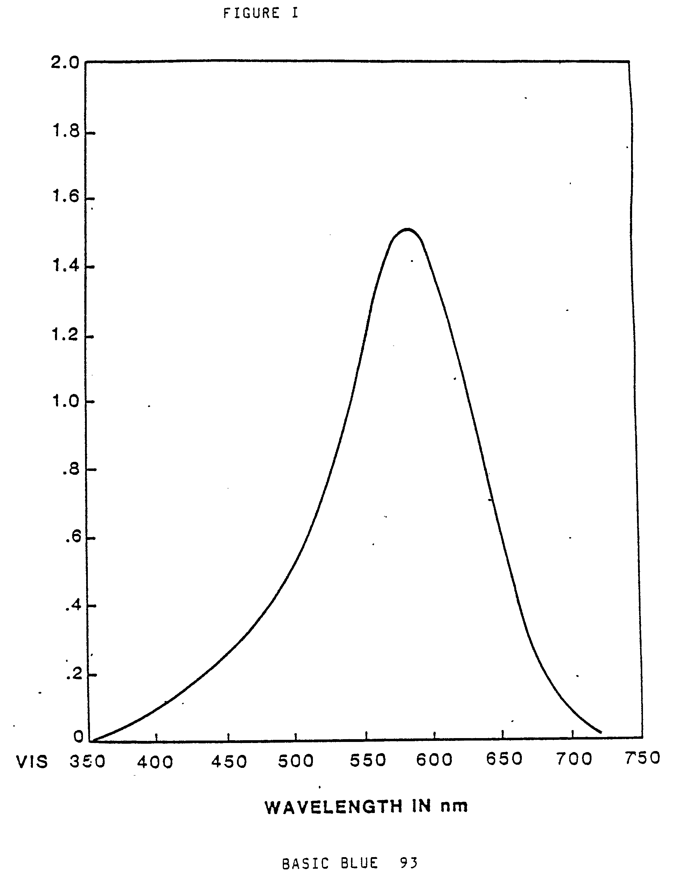

The remarkable basic cationic dye of this invention known generally as basic blue 93 has not been publicly identified by chemical structure, nor identified in the Colour Index by number. It is available under the trade name "Aizen Cathilon Navy Blue RLH" from Hodagaya Chemical Company of Japan and has the absorbance spectra recorded in Figure 1.

As a result of a comprehensive testing program of nearly 5,000 synthetic organic dyestuffs from world-wide sources, basic blue 93 has been the only dye found that preferentially and intensely stains primary granules or lysosomes in cells of the neutrophilic granulocytic series, including myeloblasts and immature granlocytic cells including promyelocytes as illustrative. Basic blue 22 or Astiazon blue FGLN is a poor second.

In reduction to practice of the invention a stock solution of the dye, basic blue 93, is dissolved in distilled water. A dye concentration of 10% has been found useful and no criticality appears to be associated with an unbuffered solution which shows no apparent deterioration or settling or evidence of change in quality over storage at room temperature for at least one full year. The color of the aqueous solution is broadly described as a reddish-purple.

A preferred laboratory procedure has been to prepare coverslips with human biopsy specimens, illustratively; blood, blood and bone marrow, imprints of bone marrow biopsy core, lymph node imprints and the like by saturation of the specimen in absolute methanol as the fixative for about two minutes. (Oddly, the stain is operative in aqueous solution when applied to dried unfixed cells. However, there is superior and preferred localization of the reaction product when the stain is applied to fixed cells).

Subsequently the fixed specimen slide preparation is rinsed in distilled water. An aqueous solution of basic blue 93 is applied to the rinsed surface of the specimen and stained for about three minutes, washed again with distilled water to remove excess dye color and mounted with Permount (or equivalent on cleaned glass slides for normal light microscope examination.

Upon examination of normal peripheral blood samples, only a few tiny black granules have been observed in normal neutrophils and bands. More granules were observed in the field with bands than were apparent with the neutrophils. Black granules as were noticed of the type found in the neutrophils were not visually apparent among the eosinophils, basophils, lymphocytes, monocytes, erythrocytes or platelets present.

Normal bone marrow aspirates stained with basic blue 93 revealed nuclei of all cells to be stained pale purple whereas the cytoplasm of the cells were less red, a pale lavender.

In cells of the neutrophilic granulocytic series, primary granules or lysosomes were stained an intense black. Promyelocytes also exhibited large numbers of granules which were stained black. In study of the myelocytes fewer numbers of granules were noted as compared with promyelocytes. Among the metamyelocytes, bands and neutrophils only a few tiny black granules were observed.

The granules in basophils and eosinophils were stained from a pale lavender to a cream color. No black granules were observed. Likewise, no black granules were found among the megakaryocytes, mast cells, plasma cells, erythroblasts and histiocytes of normal bone marrow aspirates as above.

Examination of leukemic lymphoblasts stained with basic blue 93 as above revealed both nuclei and cytoplasm to be stained a pale purple. No black granules were detected. Prominent darker purple aggregates of nuclear chromatin are observable in the nuclei of leukemic lymphoblasts stained with basic blue 93. In leukemic myeloblasts from patients with acute myeloblastic leukemia, black granules were found, their numbers varying in the cytoplasm of many of the leukemic blasts. These observations were confirmed in parallel slides where peroxidase stain and Sudan black B stains also gave similar positive confirmation. However, in some instances where the parallel and comparative slides in which Sudan black B stain and the peroxidase stain were negative in response, basic blue 93 staining was positive. The leukemic blasts were confirmed to be myeloblasts when checked out through use of monoclonal antibodies.

Leukemic blasts from patients known to suffer acute promyelocytic leukemia, stained with basic blue 93 as above, revealed large numbers of black stained granules. Auer rods, a marker for leukemic blasts of granulocytic origin, also stained black.

Biopsy specimens from patients with acute myelomonocytic leukemia, similarly stained, disclosed leukemic monocytes having a few black granules in the cytoplasm confirming the granulocytic origin of the cells. Leukemic monocytes from a patient known to suffer from acute histiomonocytic leukemia, comparatively examined, did not reveal the presence of black stained granules.

Prior art stains presently known and used to identify cells of the neutrophilic granulocytic series including, as illustrative, myeloperoxidase, Sudan black B, specific esterase and the new stains, amongst which are direct black 38 and acid blue 1, lack the following several important advantages in direct comparison to basic blue 93. These include the following:

1. Identification of both myeloblasts and promyelocytes is more accurate than heretofor due to unusually precise localization of the dye-cell reaction product in the lysosomes of immature granulocytic cells:

2. The dye-cell reaction product is jet black. It is easily visible in contrast to the pale lavender color of the nucleus.

3. Basic blue 93 differentially stains both nucleus and granules, thus a separate counterstain for the nucleus is unnecessary.

4. The subject stain is both selective and specific for primary granules (lysosomes). Cells, including myeloblasts, promyelocytes and myelocytes containing a preponderance of these granules are more accurately differentiated and identified than heretofor.

5. Virtually no confusing background precipitates occur. The problem of delineation between reaction products and non-specific precipitation is made minimal.

6. Addition of an exogenous substrate, as in the specific esterase reaction, for example, or addition of oxidizing agents, illustratively hydrogen peroxidase, as in the myeloperoxidase reaction, is no longer required.

7. Dye couplers, which require pre-sensitization before use, illustratively hexazotization of a coupler in the specific esterase reaction, are no longer needed.

8. The dye-specimen reaction product shows no detectable fading with time (tests over a year).

9. The dye is applicable to the immediate and rapid diagnosis of acute leukemia where delay in institution of treatment may be detrimental. This contrasts with use of the prior art specific esterase reaction where incubation may require as long as thirty minutes.

10. Delineation of nuclear chromatin in the identification of leukemic lymphoblasts is made more specific and definite.

The following examples are illustrative of comparisons in diagnostic studies of patients using prior art dyes along with parallel comparisons using basic blue 93. In all cases fixed biopsy specimens were compared.

Example 1

A 40 year old white male was admitted to the hospital with fever and chills. On physical examination, he had normal vital signs. The liver and spleen were enlarged. Laboratory values included hemoglobin 8.2 g%, white blood cell count 135,000/mm$^3$, and platelet count 32,000/mm$^3$.

On Wright's stain of the peripheral blood and bone marrow, many of the cells were leukemic blasts with delicate nuclear chromatin pattern and basophilic cytoplasm devoid of granularity.

Using Sudan black B and myeloperoxidase staining, a few of the leukemic blasts showed activity of peroxidase and faint staining with Sudan black B.

Using basic blue 93, many of the leukemic blasts contained black punctate granules in the cytoplasm indicating more clearly their granulocytic origin than was manifest in the use of Wright's stain and Sudan black B.

Diagnosis made: Acute myeloblastic leukemia.

Example 2

A 38 year old black female was admitted to the hospital because of nosebleeds. On physical examination, her vital signs were normal. The patient had slightly enlarged lymph nodes in the neck and groin. Laboratory data included hemoglobin 7.1 g%, white blood cell count 83,000/mm$^3$, and platelet count 22,000/mm$^3$. Evidences of disseminated intravascular coagulation, with low level of fibrinogen, and increased levels of fibrin split products were observed.

On Wright's stain of peripheral blood and bone marrow, many leukemic blasts were seen. Some blasts contained multiple Auer rods and many granules.

Using Sudan black B and myeloperoxidase stains, a few granules and Auer rods could be recognized in the leukemic blasts.

Using basic blue 93, many granules were seen in the leukemic blasts. Granules stained in intense black. Multiple Auer rods were easily isolated and identified by characteristic shape and intense black color. More Auer rods and more granules were enumerated than with comparative use of the prior art dyestuffs above.

Diagnosis made: Acute promyelocytic leukemia.

Example 3

A 22 year old white male admitted to the hospital complaining of weakness and fatigue. Physical examination showed patient with normal vital signs. Enlarged lymph nodes and enlarged liver and spleen were detected. Laboratory values included hemoglobin 6.3 grams%, white blood cell count 5,600/mm$^3$, and platelet count 12,000/mm$^3$.

Wright's stain of his peripheral blood and bone marrow showed large numbers of leukemic blasts with coarse appearing nuclear chromatin and basophilic cytoplasm devoid of granularity.

Using Sudan black B and myeloperoxidase stains, no granules were found to be identified in the cytoplasm of the leukemic blasts and the distinctive features of nuclear chromatin could not be identified.

Using basic blue 93, no granules were seen in the leukemic blasts. Nuclear chromatin displayed a distinctive pattern of prominent lavender purple colored aggregates. None of the above stains developed a similar visible pattern of nuclear aggregates in the cytoplasm of the leukemic blasts.

Using specific monoclonal antibodies, the diagnosis of acute lymphoblastic leukemia was confirmed.

Example 4

Upon hospital admittance of a black female, aged 34, experiencing fever and pain in the right upper quadrant, physical examination reported a temperature of 101° and tenderness to palpitation in the pain area. Laboratory reports detailed homoglobin 13 gram%, white blood count 22,000/mm³ and platelets 300,000/mm³. Ultrasound gall blader examination showed multiple stones present.

Wright's stain of peripheral blood reported larger numbers of neutrophils, bands and a few metamyelocytes were reported.

On staining with myeloperoxidase, activity of the enzyme was found in all granulocytic cells, particularly promyelocytes and myelocytes.

Sudan black B confirmed staining of neutrophils was reported.

Using basic blue 93, intense black granules were found in more numerous frequency in the promyelocytes and myelocytes. A relative few granules were observed in granulocytic bands and neutrophils. The granules were, when present, more numerous and more intensely stained than with the prior art stains above. Identification of immature leukocytes was more certain with the basic blue 93 dyestain. Bone marrow examination showed no evidence for leukemia.

The diagnosis of neutrophilic leukocytosis with "shift to the left" resulted. After cholecystectomy, the patient's blood counts returned to normal.

Example 5

An oriental female, 38 years, was admitted to the hospital with a rapidly enlarging mass in the left cervical area. Physical examination established presence of 3×4 cm mass and normal vital signs. Laboratory analysis included hemoglobin 14 grams%, white blood cell count 8,500/mm³ with normal differential and platelet count 205,000/mm³. The biopsied mass proved to be an abnormal lymph node. A plurality of lymph node imprints were made.

Wright's stain indicated the presence of a large number of primitive appearing cells.

Test with stains for myeloperoxidase, specific esterase and Sudan black B established a few of the cells to contain a black reaction product suggesting their granulocytic origin.

Further checking with basic blue 93 established many of the primitive cells contained numerous black, granular reaction products more prominent than observed in the prior tests above. Identifications of immature leukocytes were more precise.

Granulocytic sarcoma (chloroma) was diagnosed. Within 4 months the patient expired with acute myeloblastic leukemia.

In the above examples the human biopsy specimens were fixed before staining with basic blue 93. However, it has also been determined that basic blue 93 is operative when applied on or to dried but unfixed cells in an aqueous environment. However, use of fixed cells provides a more sharply localized reaction product and the nuclear detail is improved. Oddly, the dye appears to act both as a fixative as well as a stain. The dye is inoperative as a supravital stain for lysosomes.

Claims

1. The use of a basic cationic dye known as basic blue 93 and which dye is further fully identified by its known absorbance spectrum for differentiation and identification of normal and abnormal cells of the neutrophilic granulocytic series whereby the reaction product of a dried and/or fixed human biopsy specimen in an aqueous environment containing at least one of the said cells in the defined series is obtained with said dye.

2. A method for differentiation of both normal and abnormal cells of the neutrophilic granulocytic series and identification of individual ones of said cells present in a dried and/or fixed human biopsy specimen which comprises reacting a basic cationic dye having the absorbance spectra of basic blue 93 in an aqueous environment with said specimen prior to analytical differential examination and identification of selected individual ones of said granulocytic cells present in said specimen.

3. The method of Claim 2, in which the identifiable cells are normal neutrophilic granulocytic cells including myeloblasts, myelocytes, promyelocytes, metamyelocytes, bands and nuetrophils.

4. The method of Claim 2, in which the abnormal cells are leukemic cells of granulocytic origin including leukemic myeloblasts and promyelocytes that contain Auer rods marking their granulocytic origin.

5. The method of Claim 2, applied to leukemia diagnosis which differentiates leukemic myeloblasts and leukemic promyelocytes by the black reaction product in their lysosomes from leukemic lymphoblasts which do not contain the said black reaction product.

6. The method of Claim 5, wherein the leukemic cells of granulocytic origin contain stained Auer rods in the black reaction product.

Patentansprüche

1. Anwendung eines basischen kationischen Farbstoffes, der als basisches Blau 93 bekannt ist und darüberhinaus durch sein bekanntes Absorptionsspektrum vollständig identifiziert ist, zur Differenzierung und Identifizierung normaler und anormaler Zellen on neutrophilen Granulozytreihen, wodurch das Reaktions-produkt einer getrockneten und/oder fixierten Human-Biopsieprobe in einer wässrigen Umgebung, die zumindest eine dieser Zellen in diesen definierten Reihen enthält, mit diesem Farbstoff erhalten wird.

2. Verfahren zur Differenzierung sowohl normaler als auch anormaler Zellen von neutrophilen Granulozytreihen und zur Identifizierung von einzelnen dieser Zellen, die in einer getrockneten und/oder fixierten Human-Biopsieprobe vorhanden sind, welches die Reaktion eines basischen kationischen Farbstoffes mit dem Absorptionsspektrun von basisch Blau 93 in wässriger Umgebung mit dieser Probe vor der analytischen Differentialprüfung und Identifizierung von ausgewählten einzelnen dieser in der Probe vorhandenen Granulozytellen umfaßt.

3. Verfahren nach Anspruch 2, worin die identifizierbaren Zellen normale neutrophile Granulozytzellen sind, die Myeloblasten, Myelozyten, Promyelozyten, Metamyelozyten, Ligamente und Nuetrophile einschließen.

4. Verfahren nach Anspruch 2, worin die anormalen Zellen Leukämiezellen mit granulozytischem Ursprung sind, die Leukämiemyeloblasten und -promyelozyten einschließen, die Auer-Stäbchen enthalten, die ihren granulozytischen Ursprung markieren.

5. Verfahren nach Anspruch 2, das zur Leukämiediagnose angewendet wird, welches Leukämiemyeloblasten und Leukämiepromyelozyten durch das schwarze Reaktionsprodukt in ihren Lysosomen aus Leukämielymphoblasten differenziert, die dieses schwarze Reaktionsprodukt nicht enthalten.

6. Verfahren nach Anspruch 5, worin die Leukämiezellen mit granulozytischem Ursprung im schwarzen Reaktionsprodukt gefärbte Auer-Stäbchen enthalten.

## Revendications

1. Utilisation d'un colorant cationique, basique, connu comme étant le bleu 93 basique—et lequel le colorant est en outre complètement identifié par son spectre d'absorbance connu—pour la différenciation et l'identification de cellules normales et anormales des la série granulocytaire neutrophile, par laquelle le produit de réaction d'un échantillon de biopsie humaine séché et/ou fixé dans un environnement aqueux contenant au moins une desdites cellules dans la série définie, est obtenu avec le dit colorant.

2. Méthode pour la différenciation à la fois des cellules normales et anormales des séries granulocytaire neutrophile et identification de celles individuelles desdites cellules présentes dans un échantillon de biopsie humaine séche et/ou fixé qui comprend la réaction d'un colorant cationique basique ayant le spectre d'absorbance du bleu 93 basique dans un environnement aqueux avec ledit échantillon préalablement à l'examen différentiel analytique et l'identification de celles individuelles sélectionnées desdites cellules granulocytaires présent dans ledit échantillon.

3. Méthode selon la revendication 2, dans laquelle les cellules identifiables sont des cellules granulocytaires neutrophiles normales incluant les myéloblastes, myélocytes, promyélocytes, metamyélocytes, bandes, et neutrophiles.

4. Méthode selon la revendication 2, dans laquelle les cellules anormales sont des cellules leucémiques d'origine granulocytaire incluant les myéloblastes et les promyélocytes leucémiques qui contiennent des batonnets d'Auer marquant leur origine granulocytaire.

5. Méthode selon la revendication 2, appliquée au diagnostic de la leucémie qui différencie les myeloblasts leucémiques et les promyélocytes leucémiques par le produit noir de réaction dans leur lysosomes à partir de lymphoblastes leucémiques qui ne contiennent pas ledit produit noir de réaction.

6. Méthode selon la revendication 5, dans laquelle les cellules leucémiques d'origine granulocytaire contiennent des batonnets d'Auer colorés dans le produit noir de réaction.

EP 0 183 716 B1

FIGURE I

WAVELENGTH IN nm

BASIC BLUE 93

1